# EUROPEAN PATENT SPECIFICATION

(11) **EP 2 931 400 B1**
(45) Date of publication and mention of the grant of the patent: **07.07.2021**
(21) Application number: 13862375.6
(22) Date of filing: 12.12.2013
(51) Int. Cl.: B01D 15/20, B01J 20/34, C07K 1/16, G01N 30/50

(54) **METHOD FOR CLEANING OF PACKED BED CHROMATOGRAPHY COLUMNS**
VERFAHREN ZUR REINIGUNG VON SCHÜTTBETT-CHROMATOGRAFIESÄULEN
PROCÉDÉ DE NETTOYAGE DE COLONNES DE CHROMATOGRAPHIE À LIT GARNI

(30) Priority: 14.12.2012 SE 1251422
(43) Date of publication of application: 21.10.2015
(73) Proprietor: Cytiva BioProcess R&D AB, 751 84 Uppsala (SE)
(72) Inventor: LACKI, Karol, S-751 84 Uppsala (SE); JOHANSSON, Hans, J., S-751 84 Uppsala (SE)
(74) Representative: Larsson, Jan Anders
(86) International application number: PCT/SE2013/051493
(87) International publication number: WO 2014/092636

(56) References cited:
- EP-A1- 1 144 067
- WO-A1-2006/126942
- WO-A1-2012/072029
- US-A1- 2011 077 766
- US-A1- 2013 248 430
- US-B1- 6 972 327
- US-B2- 7 052 609
- None

## Description

### Technical field of the invention

The present invention relates to cleaning and/or sanitization methods for chromatography matrices, and more particularly to a chromatographic separation process comprising a cleaning/sanitization step. The invention also relates to a method of cleaning and/or sanitizing a chromatography column.

### Background of the invention

Chromatography is today a well-established purification method used in the biopharmaceutical industry. Typically, a liquid feed containing a target biomolecule is passed through a column containing a packed bed of matrix particles (also called resin, media etc.) to which ligands have been chemically coupled. The target biomolecule binds to the ligands on the matrix and can be recovered in purified form after desorption with an elution buffer. Alternatively, certain impurities bind, while the purified non-bound target molecule can be recovered in the flowthrough, i.e. the depleted liquid that has passed through the column.

Chromatography matrices are costly and are hence normally reused for purification of subsequent batches of the target biomolecule. To enable reuse the matrix is cleaned in situ in the column, typically with an alkaline cleaning solution such as 1 M NaOH. The alkali causes desorption of strongly bound contaminants and hydrolyses proteinaceous contaminants. This process is called cleaning in place, abbreviated as CIP. Media with proteinaceous ligands such as the Protein A media commonly used for monoclonal antibody processing offer some special cleaning challenges in that they are more costly than other media and there is thus a strong incentive to use them for many cycles. On the other hand the proteinaceous ligands are more or less alkali sensitive, which sets limits on the NaOH concentration in the cleaning solution. New alkali stable Protein A varieties have been developed, see e.g. US Pat No 8,198.404, which allows the use of NaOH solutions up to 0.5 M concentration for cleaning, but the cleaning efficiency is still somewhat lower than for the 1 M solutions commonly used with e.g. alkali-stable ion exchange matrices. Different cleaning solutions for this purpose have been suggested, see e.g. WO 2006/126942, US 7,052,609, WO 2000/33935, US 2013/0248430 and US 6,972,327, but they do not ensure complete removal of all material.

Although CIP with alkaline cleaning solutions generally allows the reuse of matrices, there is an issue with remaining contaminants after CIP. This in particular concerns the inlet part of the bed, where fouling often occurs due to strong adsorption of contaminants and to local precipitation of dissolved material. The fouling reduces the accessibility of the CIP cleaning solution to the matrix particles in this region and the precipitated material is often difficult to dissolve.

Accordingly there is a need for improved cleaning procedures, in particular procedures to be used between different cell culture batches.

### Summary of the invention

One aspect of the invention is to provide a separation process where a separation matrix can be reused for multiple cell culture batches with high matrix purity and low bioburden. This is achieved with a process as defined in claim 2.

One advantage is that the enhanced cleaning provides a matrix with high purity and sanitization degree. Further advantages are that the process ensures cleaning and/or sanitization of the entire matrix material, including any fouled top layer of the consolidated bed and that a stratified bed with improved separation efficiency can be created.

A second aspect of the invention is to provide a method for efficient cleaning and/or sanitization of a chromatography column. This is achieved with a method as defined in claim 1.

Further suitable embodiments of the invention are described in the dependent claims.

### Brief description of drawings

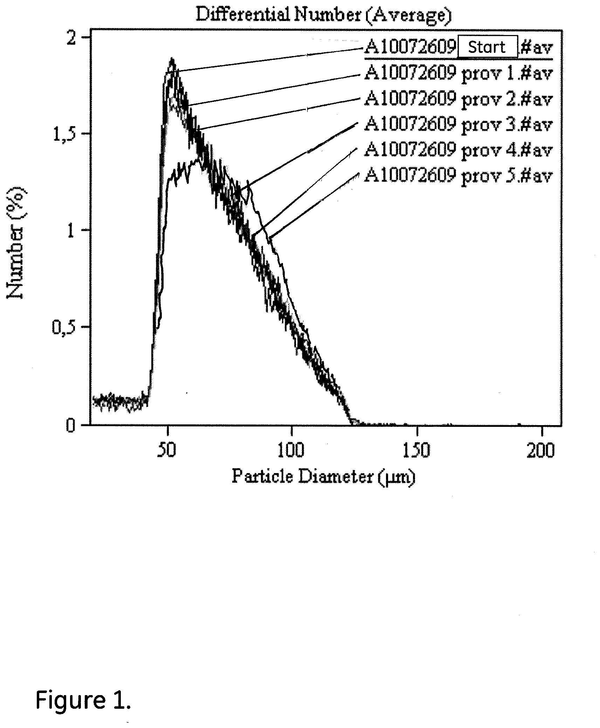

**Figure 1****.** Differential Number (Average) plot including particle size analysis of five bed cross sections (1 = settled bed top, 5 = settled bed bottom) from a repacked bed according to the invention. A10072609 Start.#av is a reference analysis of the agarose matrix lot before the column experiments.

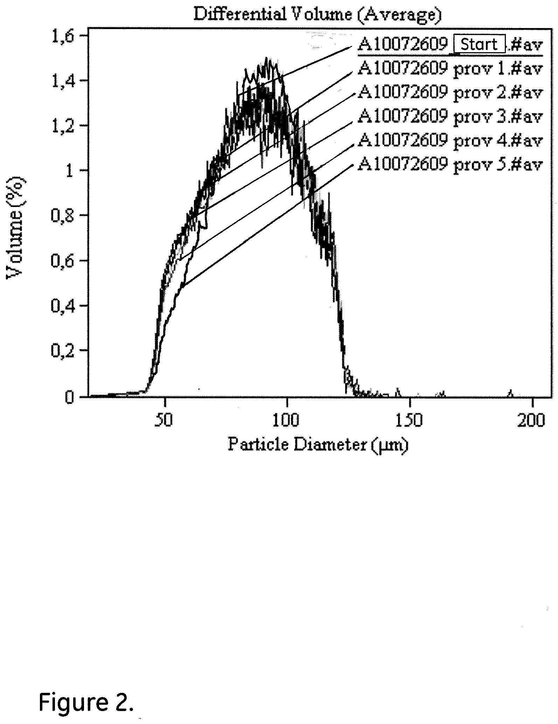

**Figure 2****.** Differential Volume (Average) plot including particle size analysis of five bed cross sections (1 = settled bed top, 5 = settled bed bottom) from a repacked bed according to the invention. A10072609 Start.#av is a reference analysis of the agarose matrix lot before the column experiments.

### Definitions

The expression "consolidated bed" herein means a bed of particles with a volume fraction of particles high enough that the bed behaves as a solid or a plastic (deformable) solid. In rheological terms this may be expressed such that the bed has a yield stress higher than about 100 Pa. In terms of chromatographic properties it means that the bed is stable enough that liquids can be conveyed through the interstitial volume between the particles without disrupting the bed structure.

The expression "liquefied bed" herein means that the volume fraction of particles is low enough that the bed behaves as a liquid or slurry. In rheological terms this may be expressed such that the bed has a yield stress lower than about 50 Pa. In terms of handling properties it means that the bed is pourable and pumpable. In an analogous manner, the expression "liquefy the bed" herein means the action of converting the bed to a liquefied bed.

### Detailed description of embodiments

In a first aspect the present invention discloses a process for chromatographic separation of at least one target biomolecule from at least one contaminant. The process comprises the steps of:
a) providing an axial chromatography column comprising a consolidated bed of separation matrix particles, said consolidated bed being confined between a bottom support net and a movable top adaptor;
b) separating a target biomolecule from at least one contaminant on the column;
c) raising the adaptor by at least 10% of the height of the consolidated bed;
d) flowing a first cleaning liquid upwards through the bed under conditions sufficient to liquefy the bed, and;
e) repacking the matrix particles of the liquefied bed to create a consolidated bed and lowering the adaptor such that it contacts the packed bed and optionally compresses it, and;
f) separating a target biomolecule from at least one contaminant on the column.

The column can be any axial column with a movable top adapter, such as e.g. the Axichrom™, Chromaflow™, BPG or XK columns (GE Healthcare), but for automation of the process it is advantageous if the adaptor is hydraulically moveable such as in the Chromaflow and Axichrom columns. The matrix particles may comprise ligands such as affinity ligands, ion exchange ligands, multimodal ligands, chelating ligands etc. The target biomolecule(s) may e.g. be proteins, peptides, nucleic acids or virus particles. They can e.g. be antibodies, antibody fragments, antibody fusion proteins, vaccine antigens, insulin etc. The target biomolecules can be applied to the column as crude feeds, e.g. clarified cell culture supernatants or plasma fractions, or they can be applied in semipurified form, e.g. as eluates from a previous chromatography step. The target biomolecule in step f) may be the same as the target biomolecule in step b), but it can also be different, such that a first target biomolecule species is separated in step b), while a second, different, target biomolecule species is separated in step f). The contaminants to be removed in steps b) and f) can be non-product-related, such as host cell proteins, DNA, endotoxins, viruses, cell culture media components etc., product-related such as aggregates, misfolded or otherwise inactive species, fragments, isoforms etc. or process-related such as leached protein ligands, virus inactivation chemicals, buffer components, modification reagents etc. The separations in steps b) and f) may be in the form of bind-elute separations, i.e. that the target biomolecules adsorb on the matrix particles and are then desorbed/eluted with elution buffers. Alternatively, the separations may be in the form of flowthrough separations, where contaminants adsorb to the matrix particles and the target biomolecules are recovered from the flowthrough fractions. The separations can further be conducted in an intermediate mode with target biomolecules recovered both from the flowthrough fractions and wash/elution solutions, e.g. as described in WO 2006/99308.

The raising of the adaptor is done to give sufficient space for liquefaction of the bed, as the volume fraction of particles in the bed will have to be increased to allow liquefaction. The adaptor can e.g. be raised by at least 10% or 15%, such as at least 25% or at least 35%, 10-250%, 10-35%, 10-25%, 15-250%, 15-35%, 100-250% or 25-250% of the height of the consolidated bed. A small raise of the adaptor means that the volume of the liquefied bed is low, and in consequence the amount of cleaning liquid can be kept low. On the other hand, the upward flow rate will then have to be carefully controlled to counteract both sedimentation and collection of matrix particles towards the adaptor. Higher raises make it easier to control the flow rate, but require higher amounts of cleaning liquid. This can to some extent be counteracted by recirculation of the cleaning liquid, possibly via a filter. The adaptor raising can be made by external force, such as a hydraulics system, but it can also be made by the flow of the cleaning liquid in step d). In this case, steps c) is performed as a part of step d) by releasing the adaptor so it can move freely in a vertical direction, closing the outlet from the adaptor and flowing the cleaning liquid upwards so that the adaptor moves to the desired height. The adaptor can then be locked into position, the outlet opened and step d) continued. Regarding step d), the skilled person will appreciate that some amount of experimentation or simulation (e.g. using the Richardson-Zaki equation described below) may be needed to determine the best conditions for liquefaction of the bed, particularly concerning the flow velocity profile. The experimentation can be facilitated if the column sidewall is transparent, which allows direct observation of the liquefaction.
The repacking of the bed and the compression can be made using standard methods for column packing, e.g. as described in the manufacturer's instructions for the particular matrix used. The repacking can e.g. be made by sedimentation, by flowing a packing fluid downwards through the column or by pushing the adaptor downwards to force the matrix particles into a consolidated bed. The packing result can also if desired be evaluated by well-known column efficiency (plate number) tests.

During bioprocessing use of packed bed columns, the top layer of the bed tends to be subjected to fouling. This is due both to irreversible adsorption of feed components on the beads and to precipitation or filtration of insoluble components in the feed by the beads in the top layer. As the fouling increases, the interstitial pores between the beads can decrease in size, leading to an increased filtration effect and accelerated fouling. This fouling is particularly difficult to remove by conventional CIP, but when the bed is broken up in the liquefaction procedure, all the bead surfaces become accessible and release of colloidal material is facilitated by the mechanical action of the process. This applies in particular to the bead-bead contact zones, where diffusion into and out of the beads is limited already in a clean bed and since contaminants will predominantly deposit around these zones, the mass transport is severely limited there in a fouled packed bed. By breaking up the bed, the former contact zones will be accessible for diffusion and the hydrodynamic forces will further contribute to removal of the fouling layer. Additionally, an improved cleaning of the column hardware surfaces is achieved.
During repacking, the former top layer beads will also be distributed throughout the bed, preventing fouling acceleration effects of any remaining colloidal material.

Further, repacking the bed leads to a certain degree of stratification of the bed, with larger beads enriched at the bottom and smaller at the top. This can be used to advantage if, during the separation of step f), the flow is in an upward direction. The target biomolecule will then first meet large beads and then progressively smaller beads, which provides for more efficient mass transport as described in M Li, A Liapis: J. Sep. Sci. 35, 947-956, 2012. Stratification will be facilitated if the adaptor is raised to a high position, e.g. by 100-250% of the consolidated bed height, which means that the repacking will be made from a low matrix particle concentration.

The target biomolecule(s) may e.g. be proteins, peptides, nucleic acids or virus particles. They can e.g. be antibodies, antibody fragments, antibody fusion proteins, vaccine antigens, insulin etc. The target biomolecules can be applied to the column as crude feeds, e.g. clarified cell culture supernatants or plasma fractions, or they can be applied in semipurified form, e.g. as eluates from a previous chromatography step. The target biomolecule in step f) may be the same as the target biomolecule in step b), but they it can also be different, such that a first target biomolecule species is separated in step b), while a second, different, target biomolecule species is separated in step f). The separations in steps b) and f) may be in the form of bind-elute separations, i.e. that the target biomolecules adsorb on the matrix particles and are then desorbed/eluted with elution buffers. Alternatively, the separations may be in the form of flowthrough separations, where contaminants adsorb to the matrix particles and the target biomolecules are recovered from the flowthrough fractions. The separations can further be conducted in an intermediate mode with target biomolecules recovered both from the flowthrough fractions and wash/elution solutions, e.g. as described in WO 2006/99308.

In some embodiments, the process further comprises after step b) a step b') of cleaning the column by conveying a second cleaning liquid through the consolidated bed. This can be a cleaning in place (CIP) step using e.g. an alkaline cleaning solution of pH at least 10, such as at least 12, at least 13 or 13 - 14. It can typically use a 0.1 - 1 M NaOH solution. The second cleaning liquid can have substantially the same composition as the first cleaning liquid, or it can have a different composition.

In certain embodiments, the process further comprises after step f) a step f') of cleaning the column by conveying a third cleaning liquid through the consolidated bed. This can be a CIP step using e.g. an alkaline cleaning solution of pH at least 10, such as at least 12, at least 13 or 13 - 14. It can typically use a 0.1 - 1 M NaOH solution. The third cleaning liquid can have substantially the same composition as the first and/or the second cleaning liquid, or it can have a different composition.

In some embodiments, the process further comprises repeating steps b) and b') and/or steps f) and f) at least once. The process can e.g. be utilized in campaign form, with a first campaign comprising several chromatographic processing cycles of one cell culture batch, with CIP between the cycles. The column is then subjected to cleaning as described in steps c) and d) and repacked. It can then be used in a second campaign for multiple processing cycles of a second culture batch, including CIP between the cycles. The first and the second campaign can optionally involve processing of two different target biomolecules.

In some embodiments, the process further comprises a step of storing the column between steps e) and f). The column may e.g. be stored for at least one week, such as at least one month, at least three months or one week to two years, such as one week to one year. The storage temperature can e.g. be room temperature or 15 - 30°C. It is of particular importance to remove contaminants, foulants and any spores or microorganisms before storage to avoid any risk of biological growth in the column and to avoid further insolubilization of contaminants/foulants during storage.

In certain embodiments applicable to all the aspects, the process or method further comprises agitating the liquefied bed in step c). The agitation may comprise the application of an oscillating flow in step c) of the first aspect. It can also e.g. comprise vibrating or sonicating the liquefied bed. It can also comprise oscillatory movement of the adaptor up and down or it can comprise agitation by jets (e.g. liquid jets) applied through a nozzle, e.g. such as the nozzles described in US Pat 5,902,485. The agitation facilitates the liquefaction of the bed, particularly for small diameter columns and can further help releasing insoluble contaminants from the matrix particles. In severely fouled beds, the contaminants can act as a glue, holding the matrix particles together, and then the initial break-up of the bed may result in lumps of aggregated particles. It is highly desirable to disperse any such lumps by agitation, in order to enable good contact between all particles and the cleaning liquid. The oscillating flow can e.g. be performed as a periodic variation in the upward flow velocity, as pulses of upward flow alternating with periods when no flow is applied or as alternating pulses of upward and downward flow. An alternating pulse train for liquefaction of the bed may also be followed by a constant upward flow to keep the matrix particles suspended during the remainder of the cleaning cycle. Agitation by jets through a nozzle can conveniently be carried out in a column having one or more nozzles for packing and unpacking the bed. Examples of such columns are Chromaflow™ and Axichrom™, both available from GE Healthcare Bio-Sciences AB, Sweden.

In certain embodiments applicable to all the aspects, the separation matrix particles, when equilibrated with pure water, have a density of 1.0 - 1.4 g/ml, such as 1.0 - 1.1 or 1.00 - 1.05 g/ml. It is advantageous if the particles have a higher density than the surrounding liquid, but in order to facilitate the liquefaction and the repacking, the density should not be too high.

In some embodiments applicable to all the aspects, the matrix particles comprise immobilized proteinaceous ligands, such as Protein A, Protein G, Protein L, single chain camelid antibodies or functional varieties thereof. Proteinaceous ligands offer very high selectivities for the capture of commercially interesting proteins such as antibodies and antibody fragments, but their stability towards highly alkaline cleaning liquids is limited. Matrices with proteinaceous ligands are also costly, emphasizing the interest in reusing them for different processes. Hence, it is of particular interest to improve the interprocess cleaning of these matrices.

In certain embodiments applicable to all the aspects, the first cleaning liquid, and optionally also the second and/or third cleaning liquid, is alkaline, such as with a pH value of at least 10, at least 12, at least 13 or 13 - 14. Alkaline cleaning liquids are usually the most effective ones, with cleaning efficiency generally increasing with pH, although the stability of the matrix in many cases set an upper limit to the useful pH range. Alkaline cleaning liquids may be aqueous solutions of NaOH, KOH etc., e.g. with NaOH or KOH concentration of 10 mM-5M, such as 10 mM-1 M or 10 mM-0.1 M, but they may also comprise other components such as water-miscible solvents, surfactants, chaotropes, reducing agents, salts, disinfectants etc. The cleaning liquid may also provide sanitization of the column, i.e. the killing of microorganisms or spores present in the bed or elsewhere in the column. This can be achieved by the alkali, but it is also possible to use other disinfectants like e.g. hypochlorite, chlorine dioxide, hydrogen peroxide or peracetic acid in cases where the matrix and the column are compatible with these agents. In some embodiments, also applicable to all the aspects, one or more of the cleaning liquids is acidic, e.g. comprising one or more of phosphoric acid, acetic acid, benzyl alcohol and/or peracetic acid.

In some embodiments applicable to all the aspects, the inner diameter of the chromatography column is at least 2.5 cm, such as at least 10 cm, at least 20 cm, 2.5 - 200 cm, 10-200 cm or 20-200 cm. It is easier to liquefy consolidated beds with higher diameters. Further, the process is particularly advantageous for pilot and full scale manufacturing of biopharmaceuticals, where columns of large diameters are normally used.

In certain embodiments applicable to all the aspects, the height of the consolidated bed is 5 - 50 cm, such as 5-40 or 5-30 cm. The liquefaction is again easier if the bed height does not exceed 30-50 cm. On the other hand, repacking is easier if the bed height is at least 5 cm, particularly in columns of larger diameters. Stratification of the repacked bed is facilitated if bed heights of 10 - 50, such as 10 - 40 cm are used.

In some embodiments applicable to all the aspects, the ratio between the inner diameter of the chromatography column to the height of the consolidated bed is at least 0.4, such as 0.4 - 40, 1-40, 1-20, 5-40, 5-20, at least 0.4, at least 1 or at least 5. A high diameter/height ratio makes it easier to liquefy the bed, but repacking is facilitated if the ratio is not too high.

In certain embodiments applicable to all the aspects, the matrix particles are essentially spherical, with (volume-weighted) average diameter at least 20 µm, such as 30 - 300 µm, 30 - 150 µm or 50 - 100 µm. Both liquefaction and repacking are facilitated if the average bead diameter is 20 µm or higher and it is advantageous not to exceed 300 µm to avoid overly high sedimentation rates. Also, the spherical shape facilitates liquefaction and repacking.

In some embodiments applicable to all the aspects, the (volume-weighted) coefficient of variation of the matrix particle size distribution is 10 - 50 %, such as 10 - 40 % or 15 - 35 %. A reasonable polydispersity of the matrix particles allows for a desirable stratification of the bed during repacking. On the other hand, a too high polydispersity will give rise to excessive back pressures during operation of the column.

In certain embodiments applicable to all the aspects, the matrix particles comprise a polysaccharide support, such as crosslinked agarose. Polysaccharide matrices are hydrophilic and resilient which facilitates liquefaction and repacking. They are also non-brittle, which diminishes the risk of attrition during the cleaning and repacking operations. Further, they have suitable densities for these operations. In some embodiments, polysaccharide supports free from high density fillers are used. Such supports will have densities in the 1.0 - 1.05 mg/ml range.

In some embodiments, the upward flow velocity in step d) is 100 - 500 cm/h, such as 200 - 400 cm/h and the viscosity of the first cleaning liquid is 1.0 - 1.5 mPas, such as 1.0 - 1.3 mPas. The velocity and viscosity affect the liquefaction and the suspension of the matrix particles during cleaning and the skilled person will appreciate that some experimental work may be needed to find the optimal velocity for a given liquid viscosity. Alternatively, suitable flow velocities can be calculated using e.g. the Richardson-Zaki equation u = uᵢεⁿ, where u is the settling velocity for particles in a suspension of voidage (volume fraction of liquid) ε. uᵢ is the unhindered settling velocity for the particles at infinite dilution and n is a coefficient selected depending on the Reynolds number Re of the system according to Re<0.2: n = 4.65 + 19.5 d/D; 0.2<Re<1: n = (4.35 + 17.5 d/D) Re^{-0.03}; 1<Re<200; n = (4.45+18 d/D) Re^{-0.1}; 200<Re<500: n = 4.45 Re^{-0.1} ; Re>500: N = 2.39, where d is the average particle diameter and D is the inner diameter of the column. uᵢ can be calculated from Stokes' law: ui= g d² (ρₚ - ρₗ)/18 η, where g is the gravitational acceleration, ρₚ is the particle density, ρᵢ the liquid density and η the liquid viscosity. The upward flow velocity can suitably be selected such that it is in the same range as u.

In certain embodiments applicable to all the aspects, the process or method further comprises, between steps d) and e), a step of removing a sample of the cleaned matrix particles and analyzing the sample with respect to purity. This step ensures that a sufficient matrix purity has been achieved by the cleaning step. If the column has a high volume, e.g. at least 1 L or at least 10 L, the removal of a small sample (e.g. less than 1 mL or less than 10 mL) will not be detrimental to the subsequent process steps. Depending on the type of matrix and the contaminant profile, several different analysis methods can be applicable, such as amino acid analysis, nitrogen analysis, Raman or IR spectroscopy etc.

In some embodiments, the process further comprises, after step f), a step of recovering the target biomolecule and, optionally after further purification, using it in a pharmaceutical formulation. If the target biomolecule in step f) is a second target biomolecule, different from a first target biomolecule in step b), this could e.g. mean that the maximum carryover of residual first target biomolecule would be 0.8 mg/L packed bed. Overall, the purity requirements for pharmaceutical use are very high, and robust elimination of any contaminants in the matrix is essential.

In a second aspect the present invention discloses a method for cleaning and/or sanitization of a packed bed chromatography column, comprising the steps of:
a) providing an axial chromatography column comprising a consolidated bed of separation matrix particles, said consolidated bed being confined between a bottom support net and a movable top adaptor;
b) raising the adaptor by at least 10% of the height of the consolidated bed;
c) flowing a cleaning liquid upwards through the bed under conditions sufficient to liquefy the bed, and;
d) repacking the matrix particles of the liquefied bed by flowing a packing fluid downwards through the column to create a consolidated bed and lowering the adaptor such that it contacts the packed bed and optionally compresses it.
This method will achieve both cleaning of the matrix particles and of the column hardware.

Embodiments discussed above are also applicable to this aspect. Note that steps b), c) and d) in the second aspect correspond to steps c), d) and e) respectively in the first aspect. Note that steps b) and b') in the first aspect will correspond to steps a') and a") in the second aspect.

### Examples

### Materials / Investigated units

Column: XK-26/20 (26 mm inner diameter), article no. 28-9889-48 from GE Healthcare. Chromatography system: Äkta Avant A25-32105, GE Healthcare containing Unicorn 6.1 software.
Multisizer 3, AD48164, Beckman Coulter AB (particle size distribution analyses)

### Protein separation medium/Chemicals

Agarose matrix: High rigidity crosslinked agarose beads prepared according to the method described in US Pat. 6,602,990. The average bead diameter was 80 µm and the porosity as determined by inverse size exclusion chromatography with dextrans as probe molecules corresponded to a K_{D} value of 0.54 for dextran of molecular weight 110 kDa, meaning that 54% of the bead volume was available to the dextran molecules. The method for porosity determination is described in L Hagel et al: J Chromatogr A 743, 33-42 (1996).
0.1 M NaOH, Titrisol® from Merck, Germany.
Red dye: Procion Red, H-E7B from Drystar Textilfarben, Germany.
Blue dye: Procion Blue, H-EGN 125 from Drystar Textilfarben, Germany.

### Methods

### General gel washing procedure and determination of gel content in slurry

Gel slurry Agarose matrix containing 20% ethanol was repeatedly washed with Milli-Q water on a glass filter (Schott Duran 4) using under pressure. The semi-dried matrix was then transferred to a measuring cylinder and Milli-Q water was added to an approximate 50/50 (v/v) gel/water ratio. For packing studies, Milli-Q water was either added to or aspirated from the settled matrix slurry, in such a manner that an exact 50% (v/v) of sedimented matrix concentration was achieved.

### Filling of 50% matrix slurry in XK-26/20 column prior to packing

The sedimented slurry in the measuring cylinder was shaken to a homogeneous suspension. The open end of the cylinder was covered with Parafilm during this procedure. Then, exactly the needed volumes were added to the column.

### Particle size analyses

The particle size distributions were measured on a Malvern Mastersizer laser diffraction instrument. The data were plotted both as number and volume distributions.

### Summary of the sample preparations

The samples were suspended in 0.9% NaCl at room temperature for approximately 3 days to ensure that the beads had attained a stable particle size. The sample (g) /NaCl-volume ratios were as follows:

| Sample (cross sections) | Sample (g) /NaCl-volume ratios |
|---|---|
| 1 | 0.73 g sample + 22 mL NaCl-solution |
| 2 | 0.84 g sample and NaCl-solution to 20 mL |
| 3 | 0.83 g sample and NaCl-solution to 20 mL |
| 4 | 1.25 g sample and NaCl-solution to 30 mL |
| 5 | 1.81 g sample and NaCl-solution to 40 mL |

### Results and discussion

### Non-colored matrix

### Packing

The goal was to achieve a 60 mm high, compressed, settled bed (60 mm height equals 32 mL). For this purpose, 70 mL 50% matrix slurry was poured into the column (upper end piece removed). After filling the column, the upper end piece was mounted and Milli-Q water was pumped downwards at 25 mL/min (283 cm/h). The packing continued for 10 minutes. The column backpressure was monitored to 3. 5 bar (upper limit for XK 26/20 is 5 bar). After finishing the packing, the upper end piece was lowered until it reached the bed surface. After that the bed was further compressed by pushing the packed bed with the upper adaptor for additional 5 mm. The final height of the compressed bed was 57 mm (30 mL matrix).

### Unpacking

For the unpacking procedure, the flow direction was changed to upwards in the Unicorn software. Ideally, the unpacking flow rate should be 1 column volume (CV)/min using 0.1 M NaOH as solvent (CIP solution). One column volume (with respect to the packed matrix) equals 30 mL/min (339 cm/h). However, the upper flow rate for Äkta Avant is 25 mL/min (283 cm/h). Therefore, this was the flow rate of choice. In the unpacking protocol, the procedure was as follows:
- Loosening of upper end piece nut
- Blocking the upper end piece spring with a paper clip, thereby enabling the free movement of the upper end piece
- Starting of pump
- Stopping of pump when the upper end piece reaches the desired position
- Tightening of upper end piece nut
- Removal of paper clip (the upper end piece is entirely locked)

Upon applying flow in the upward direction, the upper end piece slowly raised to the desired position which in total encloses 67 mL. The entire packed bed rises during the unpacking. After finishing this procedure, it was noticed that after 2 minutes, the packed matrix bed started to disintegrate. After approximately 40 minutes, the matrix had settled in the bottom of the column. 5.1.3 Flow oscillation

In order to speed up bed disintegration, the flow direction can be changed in oscillating manner. Ideally, the oscillation should be performed by altering upward and downward flows using 0.1 M NaOH at a flow rate of 25 mL/min (283 cm/h). The pumping system used had a certain reaction time for changing the flow direction. That is, it took approximately 15 sec to reach the flow rate of choice. It was noticed in the development phase that it was advantageous to use longer upward time periods compared with the downward time periods. The reason for this is that when the matrix is packed in the bottom of the column (downward flow direction), the matrix "plug" is not as easily disintegrated as in the opposite direction. The final oscillating protocol included 6 combined up and down flow cycles. Each cycle contained an up flow period of 2.5 minutes followed by a down flow period of 1 minute. The total time for the oscillating protocol was 23.5 minutes.

### Test with colored resin

To prove efficiency of the mixing protocol developed, studies with dyed matrices were performed

### Dyeing and gel slurry preparation

Approximately 500 mL agarose matrix containing 20% ethanol was dyed with either red or blue dye. Two prepared matrices (red and blue dyes) were repeatedly washed with Milli-Q water on two glass filters (Schott Duran 4) using under pressure.

The semi-dried colored matrices were placed in two measuring cylinders (each 100 mL). Milli-Q water was added to each cylinder to an approximate 50/50 matrix/water ratio. Before packing, to the settled matrices, Milli-Q water was added in order to achieve one blue and one red colored 50% (v/v) matrix slurry.

### 5.2.2 Packing (three layers)

The previously packed non-colored matrix bed had a height of 57 mm. To achieve this bed height, 70 mL 50% matrix slurry was needed. Preferably, the three-colored bed should comprise three layers, including one blue layer at the bottom, one non-colored layer in the middle and finally, a red layer on the top. For each layer, 23 mL slurry was used and the packing protocol described above was followed. Milli-Q water was the packing liquid of choice. The packing procedure was followed by lowering of the upper end piece until contact with the gel surface. Finally, the adaptor was pushed mechanically 5 mm deeper into the column.

### 5.2.3 Unpacking

Unpacking was performed using 0.1 M NaOH at a flow rate of 25 mL/min (283 cm/h). The procedure was the same as for the non-colored bed, that is enabling the upper end piece to move freely upwards in the flow direction until it reached the preferred upper end position. At this point, the pump was stopped and the end piece was locked.

### 5.2.4 Oscillation

By using the oscillation protocol, the colored bed was disintegrated. As for the non-colored bed, the oscillation was performed using upward and downward pumping at 25 mL/min flow rates. The oscillation liquid of choice was 0.1 M NaOH.

### 5.2.5 Removal and analysis of settled bed

After the oscillation procedure, the purple slurry in the column was allowed to settle for one hour. The upper end piece was then lowered into contact with the bed (after release of the capillary nut by which the column was connected to the Äkta Avant). Finally, the lower end piece was removed, and by pushing the upper end piece downwards, it was possible to squeeze out the purple bed on a plastic tray. The removed bed was cut into five cross sections which were observed visually and found to be homogeneous with respect to the purple colour, showing that complete remixing of the particles had been obtained during the cleaning procedure.

### Particle size-analyses of five cross sections

The five cross sections were suspended in 0.9% NaCl. In Figures 1 and 2, the Differential Number (Average) and Differential Volume (Average) plots are shown respectively. In Table 1, the results are presented.

**Table 1.** Particle size analysis results for cross sections 1 - 5. Start is a particle size analysis of the agarose matrix lot before the column experiments.

| **Sample** | **d50vol** | **d50number** |
|---|---|---|
| Start | 85,9 | 66,5 |
| 1 | 86,7 | 67,9 |
| 2 | 86,5 | 66,8 |
| 3 | 86,0 | 67,2 |
| 4 | 88,0 | 68,3 |
| 5 | 89,1 | 72,6 |

| | | |
|---|---|---|
| Comment: The bottom cross section (no. 5) contained beads with the highest d50vol and d50number-values. | | |

## Claims

1. A method for cleaning and/or sanitization of a chromatography matrix in a packed bed chromatography column, comprising the steps of:
a) providing an axial chromatography column comprising a consolidated bed of separation matrix particles, said consolidated bed being confined between a bottom support net and a movable top adaptor;
b) raising the adaptor by at least 10% of the height of the consolidated bed;
c) flowing a cleaning liquid upwards through the bed under conditions sufficient to liquefy the bed, and;
d) repacking the matrix particles of the liquefied bed by flowing a packing fluid downwards through the column to create a consolidated bed and lowering the adaptor such that it contacts the packed bed and optionally compresses it.

2. A process for chromatographic separation of at least one target biomolecule from at least one contaminant, comprising performing steps a)-d) of the method of claim 1, and further; after step a), performing a step a') of separating a target biomolecule from at least one contaminant on the column; and
after step d), performing a step e) of separating a target biomolecule from at least one contaminant on the column.

3. The process of claim 2, further comprising after step a') a step a") of cleaning the column by conveying a second cleaning liquid through the consolidated bed and optionally repeating steps a') and a") at least once.

4. The process of any preceding claim, further comprising after step e) a step e') of cleaning the column by conveying a third cleaning liquid through the consolidated bed and optionally repeating steps e) and e') at least once.

5. The process according to any preceding claim, further comprising a step of storing the column for at least one week between steps a') and e).

6. The process according to any preceding claim, further comprising agitating the liquefied bed in step c), wherein optionally the agitation comprises:
i) vibrating or sonicating the liquefied bed or the matrix particles,
ii) agitation by jets from one or more nozzles, and/or
iii) an oscillating flow in step c).

7. The process according to any preceding claim, wherein the separation matrix particles, when equilibrated with pure water, have a density of 1.0 - 1.4 g/ml, such as 1.0 - 1.05 g/ml.

8. The process according to any preceding claim, wherein the matrix particles comprise immobilized proteinaceous ligands, such as Protein A, Protein G, Protein L, single chain camelid antibodies or functional varieties thereof.

9. The process according to any preceding claim, wherein the first cleaning liquid is alkaline, such as with a pH value of at least 10, at least 12, at least 13 or 13 - 14.

10. The process according to any preceding claim, wherein:
i) the inner diameter of the chromatography column is at least 2.5 cm, such as at least 10 cm, at least 20 cm or 10-200 cm,
ii) the height of the consolidated bed is 5 - 50 cm, and/or
iii) the ratio between the inner diameter of the chromatography column to the height of the consolidated bed is at least 0.4, such as 0.4 - 40.

11. The process according to any preceding claim, wherein the matrix particles are essentially spherical, with average diameter at least 20 µm, such as 30 - 300 µm and/or wherein the coefficient of variation of the matrix particle size distribution is 10 - 50 %.

12. The process according to any preceding claim, wherein the matrix particles comprise a polysaccharide support, such as crosslinked agarose.

13. The process according to any preceding claim, wherein the upward flow velocity in step d) is 100-500 cm/h and the viscosity of the first cleaning liquid is 1.0-1.5 mPas.

14. The process according to any preceding claim, further comprising:
i) between steps c) and d), a step of removing a sample of the cleaned matrix particles and analyzing the sample with respect to purity, and/or
ii) after step e), a step of recovering the second target biomolecule and, optionally after further purification, using it in a pharmaceutical formulation.

## Patentansprüche

1. Methode zum Reinigen und/oder Entkeimen einer Chromatographiematrix in einer Chromatographiesäule mit gepacktem Bett, das die Schritte umfasst des:
a) Bereitstellens einer Axialchromatographiesäule, die ein verfestigtes Bett aus Trennmatrixteilchen umfasst, wobei das verfestigte Bett zwischen einem unteren Trägernetz und einem beweglichen oberen Adapter eingeschlossen ist;
b) Anhebens des Adapters um mindestens 10 % der Höhe des verfestigten Betts;
c) Strömens einer Reinigungsflüssigkeit durch das Bett nach oben unter Bedingungen, die genügen, um das Bett zu verflüssigen; und
d) erneuten Packens der Matrixteilchen des verflüssigten Betts durch Strömen eines Packfluids durch die Säule nach unten, um ein verfestigtes Bett zu erzeugen, und Absenken des Adapters derart, dass er das gepackte Bett berührt und es gegebenenfalls verdichtet.

2. Verfahren zur chromatographischen Trennung mindestens eines Ziel-Biomoleküls von mindestens einer Verunreinigung, das das Durchführen der Schritte a) - d) der Methode nach Anspruch 1 umfasst; und weiter
nach Schritt a) das Durchführen eines Schritts a') des Trennens eines Ziel-Biomoleküls von mindestens einer Verunreinigung in der Säule; und
nach Schritt d) das Durchführen eines Schritts e) des Trennens eines Ziel-Biomoleküls von mindestens einer Verunreinigung in der Säule.

3. Verfahren nach Anspruch 2, das weiter nach Schritt a') einen Schritt a") des Reinigens der Säule durch Leiten einer zweiten Reinigungsflüssigkeit durch das verfestigte Bett, und gegebenenfalls das mindestens einmalige Wiederholen der Schritte a') und a") umfasst.

4. Verfahren nach einem vorstehenden Anspruch, das weiter nach Schritt e) einen Schritt e') des Reinigens der Säule durch Leiten einer dritten Reinigungsflüssigkeit durch das verfestigte Bett, und gegebenenfalls das mindestens einmalige Wiederholen der Schritte e) und e') umfasst.

5. Verfahren nach einem vorstehenden Anspruch, das weiter einen Schritt des Lagerns der Säule zwischen Schritt a') und e) über mindestens eine Woche umfasst.

6. Verfahren nach einem vorstehenden Anspruch, das weiter das Rühren des verflüssigten Betts in Schritt c) umfasst, wobei das Rühren gegebenenfalls umfasst:
i) Rütteln oder Beschallen des verflüssigten Betts oder der Matrixteilchen,
ii) Rühren durch Strahlen aus einer oder mehreren Düsen, und/oder
iii) eine oszillierende Strömung in Schritt c).

7. Verfahren nach einem vorstehenden Anspruch, wobei die Trennmatrixteilchen, mit reinem Wasser äquilibriert, eine Dichte von 1,0 - 1,4 g/ml, wie etwa 1,0 - 1,05 g/ml aufweisen.

8. Verfahren nach einem vorstehenden Anspruch, wobei die Matrixteilchen immobilisierte proteinartige Liganden, wie etwa Protein A, Protein G, Protein L, einkettige Kamel-Antikörper oder funktionelle Varietäten davon umfassen.

9. Verfahren nach einem vorstehenden Anspruch, wobei die erste Reinigungsflüssigkeit alkalisch ist, wie etwa mit einem pH-Wert von mindestens 10, mindestens 12, mindestens 13 oder 13 - 14.

10. Verfahren nach einem vorstehenden Anspruch, wobei:
i) der Innendurchmesser der Chromatographiesäule mindestens 2,5 cm, wie etwa mindestens 10 cm, mindestens 20 cm oder 10 - 200 cm beträgt,
ii) die Höhe des verfestigten Betts 5 - 50 cm beträgt, und/oder
iii) das Verhältnis zwischen dem Innendurchmesser der Chromatographiesäule zur Höhe des verfestigten Betts mindestens 0,4, wie etwa 0,4 - 40 beträgt.

11. Verfahren nach einem vorstehenden Anspruch, wobei die Matrixteilchen im Wesentlichen kugelförmig sind, mit einem mittleren Durchmesser von mindestens 20 µm, wie etwa 30 - 300 µm, und/oder wobei der Variationskoeffizient der Matrixteilchen-Größenverteilung 10 - 50 % beträgt.

12. Verfahren nach einem vorstehenden Anspruch, wobei die Matrixteilchen einen Polysaccharidträger, wie etwa vernetzte Agarose, umfassen.

13. Verfahren nach einem vorstehenden Anspruch, wobei die Aufwärtsströmungsgeschwindigkeit in Schritt d) 100 - 500 cm/h beträgt, und die Viskosität der ersten Reinigungsflüssigkeit 1,0 - 1,5 mPas beträgt.

14. Verfahren nach einem vorstehenden Anspruch, weiter umfassend:
i) zwischen Schritt c) und d) einen Schritt des Entnehmens einer Probe der gereinigten Matrixteilchen und Analysierens der Probe im Hinblick auf Reinheit, und/oder
ii) nach Schritt e) einen Schritt des Gewinnens des zweiten Ziel-Biomoleküls und, gegebenenfalls nach weiterer Aufreinigung, Verwendens desselben in einer pharmazeutischen Formulierung.

## Revendications

1. Procédé de nettoyage et/ou de désinfection d'une matrice de chromatographie dans une colonne de chromatographie à lit garni, comprenant les étapes de :
a) fourniture d'une colonne de chromatographie axiale comprenant un lit consolidé de particules de matrice de séparation, ledit lit consolidé étant confiné entre un filet de support inférieur et un adaptateur supérieur mobile ;
b) élévation de l'adaptateur d'au moins 10 % de la hauteur du lit consolidé ;
c) circulation d'un liquide de nettoyage dans un sens ascendant à travers le lit dans des conditions suffisantes pour liquéfier le lit, et ;
d) regarnissage des particules de matrice du lit liquéfié en faisant circuler un fluide de garnissage dans un sens descendant à travers la colonne pour créer un lit consolidé et abaissement de l'adaptateur pour qu'il vienne au contact du lit garni et éventuellement le comprime.

2. Procédé de séparation chromatographique d'au moins une biomolécule cible d'au moins un contaminant, comprenant la réalisation des étapes a) - d) du procédé selon la revendication 1, et en outre ; après l'étape a), la réalisation d'une étape a') de séparation d'une biomolécule cible d'au moins un contaminant sur la colonne ; et
après l'étape d), la réalisation de l'étape e) de séparation d'une biomolécule cible d'au moins un contaminant sur la colonne.

3. Procédé selon la revendication 2, comprenant en outre après l'étape a') une étape a") de nettoyage de la colonne par acheminement d'un deuxième liquide de nettoyage à travers le lit consolidé et facultativement la répétition des étapes a') et a") au moins une fois.

4. Procédé selon l'une quelconque des revendications précédentes, comprenant en outre après l'étape e) une étape e') de nettoyage la colonne par acheminement d'un troisième liquide de nettoyage à travers le lit consolidé et facultativement la répétition des étapes e) et e') au moins une fois.

5. Procédé selon l'une quelconque des revendications précédentes, comprenant en outre une étape de stockage de colonne pendant au moins une semaine après les étapes a') et e).

6. Procédé selon l'une quelconque des revendications précédentes, comprenant en outre l'agitation du lit liquéfié à l'étape c), dans lequel l'agitation comprend facultativement :
i) la vibration ou la sonification du lit liquéfié ou des particules de matrice,
ii) l'agitation par jets d'une ou plusieurs buses, et/ou
iii) un flux oscillant à l'étape c).

7. Procédé selon l'une quelconque des revendications précédentes, dans lequel les particules de matrice de séparation, lorsqu'elles sont équilibrées avec de l'eau pure, ont une densité de 1,0 - 1,4 g/ml, par exemple de 1,0 - 1,05 g/ml.

8. Procédé selon l'une quelconque des revendications précédentes, dans lequel les particules de matrice comprennent des ligands protéiques immobilisés, tels que la protéine A, la protéine G, la protéine L, des anticorps de camélidés à chaîne unique ou des variétés fonctionnelles de ceux-ci.

9. Procédé selon l'une quelconque des revendications précédentes, dans lequel le premier liquide de nettoyage est alcalin, par exemple avec une valeur de pH d'au moins 10, d'au moins 12, d'au moins 13 ou de 13 - 14.

10. Procédé selon l'une quelconque des revendications précédentes, dans lequel :
i) le diamètre interne de la colonne de chromatographie est d'au moins 2,5 cm, par exemple d'au moins 10 cm, d'au moins 20 cm ou de 10 - 200 cm,
ii) la hauteur du lit consolidé est de 5 - 50 cm, et/ou
iii) le rapport entre le diamètre interne de la colonne de chromatographie sur la hauteur du lit consolidé est d'au moins 0,4, par exemple de 0,4 - 40.

11. Procédé selon l'une quelconque des revendications précédentes, dans lequel les particules de matrice sont essentiellement sphériques, avec un diamètre moyen d'au moins 20 µm, par exemple de 30 - 300 µm et/ou dans lequel le coefficient de variation de la distribution de tailles de particules de matrice est de 10 à 50 %.

12. Procédé selon l'une quelconque des revendications précédentes, dans lequel les particules de matrice comprennent un support polysaccharidique, par exemple de l'agarose réticulée.

13. Procédé selon l'une quelconque des revendications précédentes, dans lequel la vitesse du flux ascendant à l'étape d) est de 100 - 500 cm/h et la viscosité du premier liquide de nettoyage est de 1,0 à 1,5 mPas.

14. Procédé selon l'une quelconque des revendications précédentes, comprenant en outre :
i) entre les étapes c) et d), une étape de retrait d'un échantillon des particules de matrice nettoyées et d'analyse de l'échantillon relativement à la pureté, et/ou
ii) après l'étape e), une étape de récupération de la seconde biomolécule cible et, facultativement après la purification, l'utilisation de celle-ci dans une formulation pharmaceutique.
